# EUROPEAN PATENT APPLICATION

(11) **EP 4 353 305 A1**
(43) Date of publication of application: **17.04.2024**
(21) Application number: 22201307.0
(22) Date of filing: 13.10.2022
(51) Int. Cl.: A61N 1/372, A61N 1/375, A61B 90/00

(54) **IMPLANTABLE MEDICAL DEVICE SUPPORTING AN IDENTIFICATION TAG AND METHOD FOR PRODUCING SAME**

(71) Applicant: BIOTRONIK SE & Co. KG, 12359 Berlin (DE)
(72) Inventor: Brunner, Bjoern, Portland, 97219 (US); Kramer, Kenneth M., Lake Oswego, 97034 (US); Green, Donna, ing City, 97224 (US)
(74) Representative: Biotronik Corporate Services SE

(57) **Abstract**

An implantable medical device (1) and a method for producing same are described. The medical device comprises a printed circuit board (5) which supports an identification tag (17). The identification tag comprises characters formed in a metal layer (15) comprised in the printed circuit board (5), the characters furthermore comprising a radiopaque layer (23), the radiopaque layer having a higher x-ray absorbance than the metal layer comprised in the printed circuit board. Due to the radiopaque layer, the identification tag may be visible in an x-ray image with a high contrast. The identification tag may be included in the PCB in a space saving manner and may be produced with almost no additional efforts and costs.

## Description

The present invention relates to an implantable device and to a method for producing such a device.

Medical devices may be implanted into a body of a patient. Such devices may implement various functionalities. For example, an implantable medical device may be embodied as a pacemaker.

It may be required that each implanted medical device may be uniquely identifiable. For such purpose, the medical device may support an identification tag. The identification tag may comprise information based on which the medical device may be identified and/or based on which functionalities or characteristics of the medical device may be determined. For example, the identification tag may indicate a manufacturer, a model number and/or a serial number of the medical device.

Preferably, the identification tag should be readable also when the medical device is implanted in the patient's body. For such purpose, the identification tag should be configured such that it is visible e.g. in an x-ray or fluoroscopy image and may be read-out from such image.

For example, for conventional implantable medical devices, identification tags made from tungsten or another radiopaque material are regularly used to identify the implantable medical device. These tags may frequently be embedded during an assembly process in a device header or within a housing of the device consisting for example of titanium.

US 11,020,600 B2, US 8,903,473 B2, US 2014/0257444 A1, US 2020/0121417 A1, US 8,945,090 B2, US 9,808,617 B2 and EP 3 444 007 B1 describe some conventional approaches.

The conventional approaches may suffer from various deficiencies. For example, applying an identification tag to an implantable medical device in a conventional manner may require additional space in the device, additional components for the device and/or additional processes for producing the device. This may add to costs of the device and its production. Particularly, it has been found that some modern implantable medical devices may not have conventional moulded headers and/or an inside volume of a housing may often be so densely packaged that there remains no room for conventional stamped or etched discrete metal identification tags.

It may therefore be an object of the present invention to provide an implantable medical device and a method for producing same which at least reduce some of the above mentioned deficiencies. Particularly, it may be an object of the present invention to provide an approach for an implantable medical device requiring a very small space, few components and/or few processing steps to be produced.

Such objects may be met with the subject-matter of the independent claims. Advantageous embodiments are defined in the dependent claims as well as the corresponding specification and figures.

According to a first aspect of the present invention, an implantable medical device comprising a printed circuit board is described. Therein, the printed circuit board supports an identification tag. The identification tag comprises characters formed in a metal layer comprised in the printed circuit board, the characters furthermore comprising a radiopaque layer, whereby the radiopaque layer having a higher x-ray absorbance than the metal layer comprised in the printed circuit board.

According to a second aspect of the invention, a method for producing an implantable medical device is described. The method at least comprises (i) providing a printed circuit board, (ii) generating an identification tag on the printed circuit board and (iii) integrating the printed circuit board with other components for generating the implantable medical device. Therein, the identification tag comprises characters formed in a metal layer comprised in the printed circuit board, the characters furthermore comprising a radiopaque layer, whereby the radiopaque layer having a higher x-ray absorbance than the metal layer comprised in the printed circuit board.

Ideas underlying embodiments of the present invention may be interpreted as being based, inter alia, on the following observations and recognitions.

Briefly summarised in a non-limiting manner, embodiments of the present invention relate to an implantable medical device and a method for producing same, wherein an identification tag is produced directly on a printed circuit board (PCB) included in the medical device. Accordingly, the PCB supports or carries the identification tag. Characters are formed in a metal layer comprised in the PCB for forming the identification tag. However, it has been observed that such characters formed in the PCB metal layer may have a low contrast upon being visualised in a fluoroscopy or x-ray image. Therefore, it is suggested that the characters in the PCB metal layer are "reinforced" by additionally applying a radiopaque layer on top of the characters. Particularly, it has been found that such radiopaque layer reinforcing the contrast in x-ray images may be easily applied using processes which are generally applied in SMT (surface mount technology) procedures anyway, such that no additional components, processing steps and/or equipment may be required.

The metal layer could be one single metal layer or several metal layers arranged one above the other.

In the following, characteristics of embodiments of the present invention will be described in more detail.

First, possible features, characteristics and/or advantages of the implantable medical device according to embodiments of the first aspect of the invention will be described.

The implantable medical device described herein may be miniaturized. For example, the medical device may have dimensions of only a few centimetres or less, e.g. less than 5 cm. The medical device may not comprise any separate header. Additionally or alternatively, the medical device may not comprise a separate housing, particularly no metal housing. The implantable medical devices may be an implantable pressure sensor, an implantable defibrillator, an implantable cardiac monitor (loop recorder) or an implantable pacemaker, in particular an intracardiac pacemaker (sometimes also referred to as implantable leadless pacemaker).

However, the medical device does comprise a printed circuit board. Such PCB typically supports electronic components such as resistors, capacitors, inductors, integrated circuits, etc.

Generally, the PCB comprises a substrate and a metal layer. The substrate provides mechanical stability for the PCB. The metal layer may provide for an electric contact to the electronic components and/or for electrically interconnecting the electronic components.

The substrate may consist of or comprise an electrically isolating material. Typically, the substrate consists of or comprises a plastic material such as epoxy resin. Generally, the substrate has a sheet-like or plate-like shape and has a thickness of between 0.1 mm and 10 mm, in most cases of between 0.3 mm and 3 mm.

The metal layer may consist of or comprise a highly electrically conductive metal such as copper, silver, gold or mixtures or alloys of such metals. The metal layer may comprise two or more sublayers. The metal layer typically has a thickness of between 10 µm and 1 mm, in most cases between 20 µm and 200 µm.

The PCB typically additionally comprises a cover layer covering the metal layer. The cover layer may consist of or comprise an electrically isolating material, particularly a polymer material. The cover layer typically has a thickness of between 10 µm and 1 mm, in most cases between 20 µm and 200 µm. The cover layer may be locally removed for exposing the otherwise covered metal layer. Particularly, the cover layer may be light-sensitive or have similar characteristics such that local areas of the cover layer may be removed in a photolithography process. Alternatively, the cover layer may be locally removed with other techniques such as lasering, mechanical removal, etc.

The identification tag is applied directly to the PCB, i.e. is arranged at a surface of the PCB. Preferably, the identification tag is arranged at a location of the PCB where it is not shielded or shadowed by other components of the PCB or of the entire medical device, particularly not by components having a significant x-ray absorbance.

Generally, the identification tag comprises several characters such as letters, numbers and/or symbols. The identification tag may be unique, i.e. each medical device or each type of medical device may have its individual identification tag.

The characters of the identification tag are formed in the metal layer comprised in the PCB. While this metal layer is generally used for generating electrically conductive structures for the PCB, for example in order to provide contact pads for attaching electronic components or in order to provide electrically conductive lines for interconnecting for example electronic components, it is suggested herein to locally structure the metal layer in order to thereby form the characters of the identification tag. For example, material of the metal layer may be locally removed in order to prepare a positive or negative image of letters, numbers and/or symbols.

However, while the characters formed in the metal layers may be clearly visible upon optically imaging the PCB, it has been found that such characters in the metal layer may be only visible with a low contrast in an x-ray image of the PCB.

Based on this finding, it is therefore suggested to add an additional layer on top of the metal layer for each of the characters, this additional layer having a higher x-ray absorbance than the metal layer and therefore being referred to herein as radiopaque layer. In other words, instead of forming the characters of the identification tag only with the metal layer comprised in the PCB, these characters shall be formed with a double layer including the radiopaque layer and the metal layer. Due to such double layer, the identification tag may be visible with a high contrast in an x-ray or fluoroscopy image.

According to an embodiment, the printed circuit board may support an electronic circuitry configured for controlling at least one functionality of the implantable medical device. Thus, the PCB on which the identification tag is formed may be the same PCB which supports the electronic circuitry of the medical device. Accordingly, no additional PCB has to be provided for the identification tag, but the PCB required in any case in the medical device may also be used for supporting the identification tag. As a result, space, material and costs may be saved.

Generally, the radiopaque layer obtains its high x-ray absorbance due to several characteristics, including a thickness of the radiopaque layer and material characteristics of the material(s) included in the radiopaque layer. Accordingly, these characteristics should be selected in a suitable manner for optimising a visibility of the identification tag in an x-ray image.

Particularly, according to an embodiment, the radiopaque layer may comprise a tin based mixture, in particular a mixture mainly comprising tin, silver and copper or a mixture comprising tin, bismuth and/or indium. The tin based mixtures may comprise lead as well. Such materials are generally used in PCB appliances, for example as solder for electrically contacting and fixing electronic components to metal structures in the PCB. Tin based mixtures as mentioned above as well as a tin-lead-mixtures typically have a low melting point and may therefore be liquefied at low processing temperatures. Furthermore, both materials may be easily handled as they are, inter-alia, generally non-toxic. It has been found that, with a radiopaque layer consisting of or comprising tin based mixtures and/or a tin-lead-mixture of suitable thickness, the characters of the identification tag may be visible in an x-ray image with high contrast.

According to an embodiment, the radiopaque layer has a thickness of at least 20 µm, preferably at least 100 µm. It has been found that with a radiopaque layer of too small thickness, a contrast in an x-ray image may excessively suffer, probably due to insufficient x-ray absorbance. A radiopaque layer with a sufficiently large thickness may improve the contrast in the x-ray image.

According to an embodiment, the radiopaque layer has a thickness of at most 2000 µm (2 mm), preferably at most 500 µm . It has been found that with a radiopaque layer of too large thickness, a contrast in an x-ray image may excessively suffer, probably due to excessive x-ray absorbance and/or shadowing effects. A radio back layer with a sufficiently small thickness may improve the contrast in the x-ray image.

According to an embodiment, the characters may be formed as an inverse representation with an outer metallized contour surrounding an inner non-metallized form of the characters. In other words, instead of representing a character's form with a positive image in which the character itself is represented with metallised areas, the character's form may be represented with a negative image in which the character is represented with non-metallised areas surrounded by metallised areas forming an outer metallised contour of the character. It has been found that such negative or inverse representation may result in a better visibility and/or higher contrast of the characters in an x-ray image.

According to an embodiment, the implantable medical device is an implantable leadless pacemaker. An implantable leadless pacemaker (ILP) is a miniaturised pacemaker which may be implanted directly in a patient's heart, particularly in a ventricle, where it may stimulate the heart without requiring external leads for establishing an electrical connection to an electrode. Such ILPs typically comprise an electronic circuitry mounted on a PCB but does not comprise a header. Accordingly, particularly for such ILPs, it may be beneficial to provide a unique identification using the identification tag as described herein.

Next, possible features, characteristics and/or advantages of the method according to embodiments of the second aspect of the invention will be described.

Generally, the implantable medical device described herein may be produced using various techniques and/or processing sequences.

According to an embodiment, the identification tag may be generated by (i) generating the characters in the metal layer comprised in the printed circuit board and (ii) forming the radiopaque layer by depositing radiopaque material onto the characters generated in the metal layer. In other words, for preparing the identification tag, a two-step process may be applied. Therein, in a first step, the characters are prepared in the metal layer of the PCB by suitably structuring this metal layer. Subsequently, in a second step, the radiopaque layer is formed by depositing radiopaque material onto the characters formed in the metal layer. Preferably, the radiopaque material is deposited exclusively onto a surface of the characters formed in the metal layer for generating the identification tag. The two steps may be implemented separately, i.e. using for example different techniques, different equipment, different tools, different materials, etc. Accordingly, the double layer used for forming the identification tag may be prepared with a high degree of procedural flexibility.

According to an embodiment, the characters in the metal layer may be generated using photolithography. Such photolithography is a well-established technique for structuring the metal layer in a PCB. Accordingly, as photolithography is generally used upon preparing the PCB for its use in the medical device, anyway, using photolithography additionally for generating the characters of the identification tag does generally not substantially increase a processing complexity upon producing the medical device.

Typically, in photolithography, a photosensitive layer covers an underlying layer which is to be structured. The photosensitive layer maybe locally modified by local irradiation application of light. For example, the light may be irradiated through a mask. Alternatively, the light maybe locally applied using for example a laser. After having irradiated partial areas of the photosensitive layer and having modified their characteristics thereby, these partial areas or adjacent areas may be removed such that the underlying layer, such as the metal layer, is exposed locally. Subsequently, the underlying layer may for example be locally removed in an etching procedure, thereby structuring the underlying layer.

According to an embodiment, the radiopaque layer may be formed by printing the radiopaque material onto the characters generated in the metal layer. Various printing techniques may be applied. For example, stencil printing is a well-established technique for applying materials onto a surface of a PCB. Alternatively, other printing techniques such as screen printing, roller printing, inkjet printing, etc. may be applied. As printing techniques are generally used upon preparing the PCB for its use in the medical device, anyway, using printing techniques additionally for generating the characters of the identification tag does generally not substantially increase a processing complexity upon producing the medical device.

According to an embodiment, the radiopaque layer may be formed with a solder paste. In in other words, the radiopaque layer may be formed by applying a paste containing metal particles as it is conventionally used for soldering electronic components to pads on a surface of a PCB. Using such solder paste may be an easily applicable and cost-effective way of forming the radiopaque layer.

According to an embodiment, the formed radiopaque layer may be submitted to a reflow procedure of a surface mount technique. Reflow procedures are commonly used in surface mount techniques (SMT) for mounting electronic components directly on top of a PCB. Accordingly, applying such reflow procedures also for generating the characters of the identification tag may not substantially add complexity to a procedure for producing the medical device.

It shall be noted that possible features and advantages of embodiments of the invention are described herein with respect to various embodiments of an implantable medical device, on the one hand, and with respect to various embodiments of a method for producing such medical device, on the other hand. One skilled in the art will recognize that the features may be suitably transferred from one embodiment to another and features may be modified, adapted, combined and/or replaced, etc. in order to come to further embodiments of the invention.

In the following, advantageous embodiments of the invention will be described with reference to the enclosed drawings. However, neither the drawings nor the description shall be interpreted as limiting the invention.
Fig. 1 shows a top view of an implantable medical device according to an embodiment of the present invention.
Fig. 2 shows a sectional view along the line A-A indicated in Fig. 1.
Fig. 3 shows a top view onto the identification tag in an inverse representation.

The figures are only schematic and not to scale. Same reference signs refer to same or similar features.

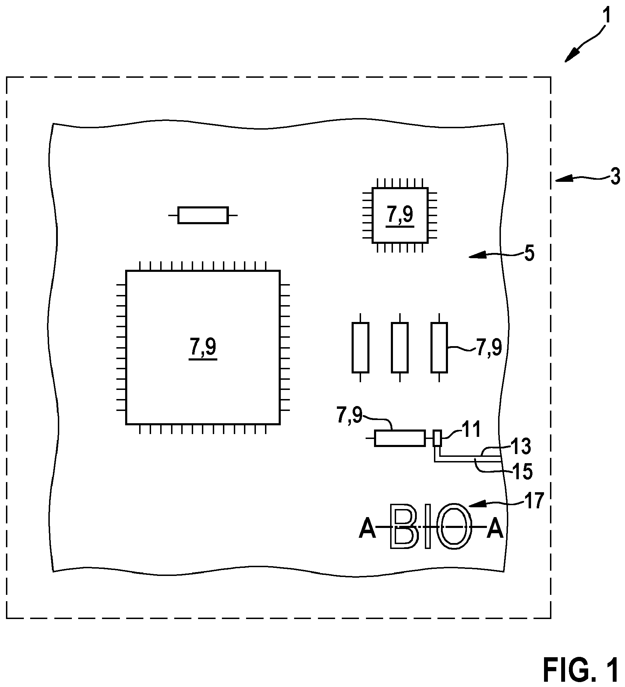

Fig. 1 shows an implantable medical device 1 symbolised by a dashed line. The medical device may be for example an implantable leadless pacemaker 3. The medical device 1 comprises a printed circuit board 5. The PCB 5 supports various electronic components 7 forming an electronic circuitry 9 configured for controlling functionalities of the medical device 1. The electronic components 7 are fixed to pads 11 and/or connected to lines 13 formed in a metal layer 15 that is included in the PCB 5 (note: for clarity reasons, only a single example of the pads and lines and a symbolised representation of the metal layer 15 are shown in Fig. 1). The metal layer 15 may comprise several sublayers and/or may comprise or consist of e.g. copper.

The PCB 5 further supports an identification tag 17. The identification tag 17 allows a unique identification of the medical device 1 (note: for clarity reasons, the identification tag is only shown in Fig. 1 as a short sequence of letters "BIO", but in reality may comprise a larger plurality of various characters).

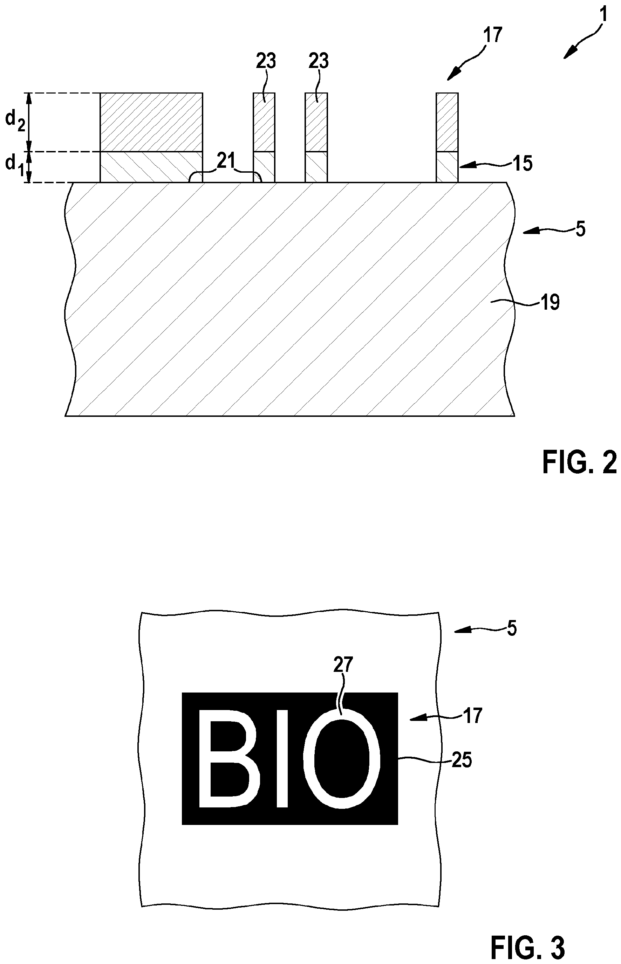

Fig. 2 shows a sectional view along a portion of the PCB 5 supporting the identification tag 17. On a surface of a substrate 19 of the PCB 5, partial areas 21 of the metal layer 15 are arranged such that they form, in a top view onto the surface of the substrate 19, characters of the identification tag 17. The metal layer 15 could be one single layer or several layers arranged one above the other. Each of the partial areas 21 is covered by additional material of a radiopaque layer 23. The radiopaque layer 23 has a higher x-ray absorbance as compared to the metal layer 15. For example, the material of the radiopaque layer 23 may absorb x-rays more effectively than the material of the metal layer 15. For example, the radiopaque layer 23 may comprise or consist of tin based mixture or a tin-lead-mixture. Particularly, the radiopaque layer 23 may be made from a solder paste. Alternatively or additionally, a thickness d₂ of the radiopaque layer 23 may be larger than a thickness d₁ of the metal layer 15. For example, the radiopaque layer 23 may have a thickness d₂ of between 100 µm and 500 µm.

Fig. 3 shows implementation of the identification tag 17 in which the characters are formed as an inverse representation. Therein, an outer metallised contour 25 surrounds in a non-metallised form 27 of the characters.

The implantable medical device 1 with its identification tag 17 may for example be produced including the following processing sequence: A PCB is provided, the PCB including a metal layer covered by a cover layer of e.g. polymer. Using photolithography, partial areas of the cover layer are selectively removed such as to expose the metal layer locally. Subsequently, the metal layer may be locally removed in the exposed areas for example using an etching process. Accordingly, only non-etched partial areas of the metal layer remained on the surface of the substrate of the PCB. Subsequently, material of the radiopaque layer may be deposited on top of the remaining partial areas of the metal layer. For example, a solder paste may be printed on top of the partial areas of the metal layer using for example a stencil printing technique. Subsequently, the PCB with its radiopaque layer formed thereon may be submitted to a reflow procedure of a surface mount technique.

The method described herein for creating radiopaque markers forming an identification tag does not consume useful volume within the medical device. This is because the volume occupied by the identification tag may be in a "dead" space between flat circuit board bridges and a curved device housing. The method does not necessarily add extra steps to a manufacturing process for producing the medical device. This is because for example copper photo etching steps are generally already required for PCB manufacturing and solder paste printing and reflow steps are already required for circuit assembly manufacturing. Furthermore, the method does not necessarily add extra cost because the copper volume of the metal layer is already present in the PCB and an additional solder volume consumed for preparing the radiopaque layer is generally negligibly small. Accordingly, the proposed method allows making smaller and/or cheaper devices than existing approaches.

Finally, it should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

### List of reference numerals

- 1: implantable medical device
- 3: pacemaker
- 5: printed circuit board / PCB
- 7: electronic components
- 9: electronic circuitry
- 11: pad
- 13: line
- 15: metal layer
- 17: identification tag
- 19: substrate
- 21: partial area of metal layer
- 23: radiopaque layer
- 25: outer metallised contour
- 27: non-metallised form of character

## Claims

1. Implantable medical device (1), comprising:
a printed circuit board (5),
wherein the printed circuit board (5) supports an identification tag (17),
wherein the identification tag (17) comprises characters formed in a metal layer (15) comprised in the printed circuit board (5), the characters furthermore comprising a radiopaque layer (23), whereby the radiopaque layer (23) having a higher x-ray absorbance than the metal layer (15) comprised in the printed circuit board (5).

2. Implantable medical device according to claim 1,
wherein the printed circuit board (5) supports an electronic circuitry (9) configured for controlling at least one functionality of the implantable medical device (1).

3. Implantable medical device according to one of the preceding claims,
wherein the radiopaque layer (23) comprises at least one of tin based mixture and a tin-lead-mixture.

4. Implantable medical device according to one of the preceding claims,
wherein the radiopaque layer (23) has a thickness of at least 20 µm.

5. Implantable medical device according to one of the preceding claims,
wherein the radiopaque layer (23) has a thickness of at most 2000 µm.

6. Implantable medical device according to one of the preceding claims,
wherein the characters are formed as an inverse representation with an outer metallized contour (25) surrounding an inner non-metallized form (27) of the characters.

7. Implantable medical device according to one of the preceding claims,
wherein the implantable medical device (1) is an implantable leadless pacemaker (3).

8. Method for producing an implantable medical device (1), comprising:
providing a printed circuit board (5),
generating an identification tag (17) on the printed circuit board (5), and
integrating the printed circuit board (5) with other components (7) for generating the implantable medical device (1),
wherein the identification tag (17) comprises characters formed in a metal layer (15) comprised in the printed circuit board (5), the characters furthermore comprising a radiopaque layer (23), whereby the radiopaque layer (23) having a higher x-ray absorbance than the metal layer (15) comprised in the printed circuit board (5).

9. Method according to claim 8,
wherein the identification tag (17) is generated by
generating the characters in the metal layer (3) comprised in the printed circuit board (5) and
forming the radiopaque layer (23) by depositing radiopaque material onto the characters generated in the metal layer (15).

10. Method according to claim 9,
wherein the characters in the metal layer (15) are generated using photolithography.

11. Method according to one of claims 9 and 10,
wherein the radiopaque layer (23) is formed by printing the radiopaque material onto the characters generated in the metal layer (15).

12. Method according to one of claims 9 to 11,
wherein the radiopaque layer (23) is formed with a solder paste.

13. Method according to one of claims 9 to 12,
wherein the formed radiopaque layer (23) is submitted to a reflow procedure of a surface mount technique.
